# EUROPÄISCHE PATENTSCHRIFT

(11) **EP 1 795 155 B1**
(45) Veröffentlichungstag und Bekanntmachung des Hinweises auf die Patenterteilung: **19.03.2014**
(21) Anmeldenummer: 07004657.8
(22) Anmeldetag: 02.07.1999
(51) Int. Cl.: A61F 2/44, A61F 2/46

(54) **Zwischenwirbelimplantat**
INTERVERTEBRAL IMPLANT
IMPLANT INTERVERTEBRAL

(43) Veröffentlichungstag der Anmeldung: 13.06.2007
(62) Teilanmeldung aus: 99931246.5
(73) Patentinhaber: Spine Solutions Inc., West Chester, PA 19380 (US)
(72) Erfinder: Beyersdorff, Boris, 10119 Berlin (DE); Marnay, Thierry, 34080 Montpellier (FR)
(74) Vertreter: Erb, Henning

(56) Entgegenhaltungen:
- EP-A1- 0 317 972
- EP-A1- 0 560 141
- EP-A2- 0 333 990
- WO-A-91/13598
- WO-A2-93/10725
- DE-A1- 3 023 353
- JP-A- 2 261 446
- US-A- 5 108 442

## Beschreibung

Die Erfindung betrifft ein Zwischenwirbelimplantat mit einem ersten Teil mit einer Außenfläche, die sich an einem ersten Wirbelkörper abstützt, und einem zweiten Teil mit einer Außenfläche, die sich an einem zweiten Wirbelkörper abstützt, wobei das erste und das zweite Teil so aneinander abgestützt sind, dass eine Beweglichkeit eines Teils relativ zu dem anderen Teil gegeben ist.

Ein derartiges Zwischenwirbelimplantat ist beispielsweise aus der US-A-5,374, 477 bekannt. Dieses Zwischenwirbelimplantat dient dem Ersetzen einer aus dem Zwischenwirbelraum entfernten Bandscheibe, und dementsprechend muss das Zwischenwirbelimplantat eine relativ geringe Bauhöhe aufweisen, da es in den Zwischenwirbelspalt hineinpassen muss. Dies ist insbesondere dann schwierig, wenn zwischen dem Oberteil und dem Unterteil noch ein zusätzlicher Gelenkeinsatz eingebettet ist, wie dies bei dem bekannten Zwischenwirbelimplantat der US-A-5,311,477 der Fall ist.

Schwierigkeiten ergeben sich aber auch schon bei zweiteiligen Zwischenwirbelimplantaten insbesondere dann, wenn diese an ihren Stützflächen noch Stifte und andere Vorsprünge tragen, die der Verankerung des Zwischenwirbelimplantates im Knochen dienen sollen. Diese können dann häufig nur dadurch eingesetzt werden, dass der Zwischenwirbelraum stark aufgeweitet wird. Dies ist nicht nur sehr schwierig, sondern birgt auch die Gefahr von Verletzungen in sich.

Da der Zwischenwirbelraum eine relativ geringe Höhe aufweist, ist es auch schwierig, an den beiden Teilen des Zwischenwirbelimplantates Angriffselemente zu befestigen, an denen ein Handhabungsinstrument angreifen kann. Es ist üblich, derartige Handhabungsinstrumente getrennt am Oberteil und am Unterteil angreifen zu lassen, beispielsweise durch Stifte, die in Bohrungen am Oberteil bzw. am Unterteil eingesteckt werden, so dass mit dem Handhabungsinstrument die beiden Teile des Zwischenwirbelimplantates in den Zwischenwirbelraum eingesetzt und gegebenenfalls auch in ihren. Abstand voneinander verändert werden können, so dass dadurch eine gewisse Aufspreizung des Zwischenwirbelraumes möglich ist. Hierzu wird beispielsweise verwiesen auf das zungenförmige Handhabungsinstrument in der US-A-5314,477. Ähnliche Implantate mit abgerundetem Bereich für eine relative Beweglichkeit sind aus der WO 93/10725A2, DE 3023353 A1, EP 0333990A2 und der EP 0560141A1 bekannt. Die EP 0317572A1 zeigt ein Implantat mit kegelstumpfartigen Verankerungen.

Aufgrund der großen Kräfte ist es notwendig, für die Angriffelemente eine gewisse Bauhöhe vorzusehen, beispielsweise müssen die Aufnahmebohrungen einen bestimmten Durchmesser aufweisen. Daraus ergibt sich eine minimale Bauhöhe für das Oberteil und für das Unterteil, und bei herkömmlichen Zwischenwirbelimplantaten addieren such somit die Bauhöhen von Oberteil und Unterteil, so dass selbst beim unmittelbaren Aufeinanderliegen von Oberteil und Unterteil eine relativ große Bauhöhe des Zwischenwirbelimplantates unvermeidlich ist.

Es ist Aufgabe der Erfindung, ein Gattungsgemäßes Zwischenwirbelimplantat so auszubilden, dass das Einführen des zweischenwirbelimplantates in den Zwischenwirbelraum erleichtert wird.

Erfindlangsgemäß wir die Aufgabe durch ein Zwischenwirbelimplantat der im beigefügten Anspruch 1 angegebenen Art gelöst. Weitere vorteilhafte Ausgestaltungen sind in den Unteransprüchen beschrieben.

Ferner kann bei einem Zwischenwirbelimplantat der beschriebenen Art vorgesehen sein, dass Oberteil und Unterteil jeweils zum anderen Teil gerichtete Vor- bzw. Rücksprünge aufweisen, die seitlich so gegeneinander versetzt sind, dass sie bei an das Unterteil angenähertem Oberteil ineinander eingreifen, und dass die Angriffselemente am Oberteil und am Unterteil jeweils in Vorsprüngen dieser Teile derart angeordnet sind, dass die Angriffselemente von Oberteil und Unterteil nebeneinander liegen und sich in Richtung der Höhe des Zwischenwirbelimplantats zumindest teilweise überlappen.

Bei einer solchen Ausgestaltung lässt sich eine minimale Bauhöhe der beiden aufeinanderliegenden zwischenwirbelimplantatsteile erreichen, da die Angriffselemente, die eine minimale Bauhöhe nicht unterschreiten können, jeweils in Vorsprüngen von Oberteil bzw. Unterteil angeordnet sind, also in den Teilen mit der größten Bauhöhe von Oberteil und Unterteil. Diese Bereiche großer Bauhöhen sind als Vorsprünge ausgebildet, neben denen sich jeweils Rücksprünge befinden, in die die Vorsprünge des jeweils anderen Teils eintauchen können. Daraus ergibt sich einmal, dass die Angriffselemente für die Handhabungsinstrumente nebeneinander liegen, und zum anderen, dass diese sich zumindest teilweise überlappen können, so dass die Gesamtbauhöhe der aufeinanderliegenden Teile des Zwischenwirbelimplantates gegenüber herkömmlichen Zwischenwirbelimplantaten deutlich herabgesetzt werden kann. Es ergibt sich somit eine verschachtelte Anordnung von Oberteil und Unterteil mit maximaler Ausnützung der zur Verfügung stehenden Materialhöhe.

Dabei ist es günstig, wenn die Angriffselemente Einstecköffnungen für stiftförmige Halteelemente eines Handhabungsinstrumentes sind, diese Einstecköffnungen können aufgrund der beschriebenen Konstruktion einen relativ großen Durchmesser aufweisen und damit kräftige Haltestifte aufnehmen, und trotzdem ergibt sich eine relativ geringe Bauhöhe des Zwischenwirbelimplantates bei unmittelbar aufeinandergelegten Teilen.

Dabei ist es vorteilhaft, wenn sich die Einstecköffnungen im wesentlichen parallel zu den Stützflächen erstrecken, auch dadurch wird vermieden, dass die Bauhöhe der Zwischenwirbelimplantatsteile vergrößert wird.

Bei einer bevorzugten Ausführungsform ist vorgesehen, dass das Unterteil eine der unteren Stützfläche gegenüberliegende zentrale Vertiefung aufweist, die von einem U-förmigen Rand umgeben ist. Die Vertiefung dient also bei unmittelbar aufeinanderliegenden Unterteil und Oberteil der Aufnahme eines Vorsprunges am Oberteil.

Dabei ist es vorteilhaft, wenn das Oberteil einen im wesentlichen komplementär in die Vertiefung passenden zentralen Vorsprung trägt, es wird also das gesamte Volumen der Vertiefung für den Vorsprung ausgenutzt.

Es ist weiterhin vorteilhaft, wenn die Angriffselemente des Unterteils an den beiden Enden des U-förmigen Randes angeordnet sind, also außen liegen.

Die Angriffselemente des Oberteils können dagegen an dem zentralen Vorsprung des Oberteils angeordnet sein, liegen also gegenüber den Angriffselementen des Oberteils weiter innen

Insbesondere können die Angriffselemente des Oberteils nahe der seitlichen Ränder des zentralen Vorsprungs angeordnet sein, so dass auch für das Oberteil der Abstand der Angriffselemente relativ groß gewählt werden kann, dadurch lässt sich das Oberteil ebenso wie das Unterteil gegen eine Verkippung zuverlässig sichern.

Bereits hier sei darauf hingewiesen, dass die Ausdrücke "Unterteil "und "Oberteil" nicht unbedingt etwas über die Einbaulage des Zwischenwirbelimplantates in der Wirbelsäule aussagen, das mit "Unterteil" bezeichnete Teil könnte in der Wirbelsäule tatsächlich auch oben liegen. Wesentlich ist lediglich, dass Oberteil und Unterteil das Zwischenwirbelimplantat auf einander gegenüberliegenden Seiten des Implantates begrenzen.

Besonders vorteilhaft ist es, wenn das Oberteil und/oder das Unterteil im wesentlichen plattenförmig ausgebildet sind, wobei diese Teile Vor- und Rücksprünge aufweisen können, die dem jeweils anderen Teil zugewandt sind. Die plattenförmige Ausbildung führt aber insgesamt zu einer sehr geringen Bauhöhe des Zwischenwirbelimplantates.

Bei einer bevorzugten Ausführungsform weisen sowohl das Unterteil als auch das Oberteil je eine Aufnahme für einen Gelenkeinsatz auf. Dieser Gelenkeinsatz, der nach dem Einsetzen des Zwischenwirbelimplantates zwischen Oberteil und Unterteil platziert wird, stützt Oberteil und Unterteil gegeneinander ab, er übernimmt beispielsweise eine federnde Funktion und führt außerdem zu einer gewissen Verschwenkbarkeit der beiden Teile eines Zwischenwirbelimplantates gegeneinander, so dass damit auch eine Verschwenkbarkeit der benachbarten Wirbelkörper erreichbar ist.

Insbesondere ist es vorteilhaft, wenn der Gelenkeinsatz mindestens eine kugelige Stützfläche aufweist, die in die entsprechend kugelig geformte Aufnahme eingreift.

Günstig ist es, wenn die kugelige Aufnahme in einem zentralen Vorsprung des Oberteils angeordnet ist.

Es ist weiterhin vorteilhaft, wenn die zentrale Vertiefung des Unterteils die Aufnahme für den Gelenkeinsatz bildet.

Gemäß einer bevorzugten Ausführungsform der Erfindung ist vorgesehen, dass der Gelenkeinsatz von der Seite in die Aufnahme einschiebbar ist, die die Angriffselemente für ein Handhabungsinstrument trägt. Es handelt sich dabei um die Seite, von der Oberteil und Unterteil in den Zwischenwirbelraum eingeführt werden, und von dieser Seite her kann dann auch der Gelenkeinsatz zwischen die bereits eingesetzten Teile des Zwischenwirbelimplantats eingeschoben werden.

Dabei ist es günstig, wenn der Gelenkeinsatz längs einer Führung in die Aufnahme einschiebbar ist.

Auch der Einsatz ist vorzugsweise im wesentlichen plattenförmig ausgebildet.

Eine besonders günstige Ausgestaltung ergibt sich, wenn der Einsatz die zentrale Aufnahme im wesentlichen vollständig ausfüllt und mit der kugeligen Stützfläche aus der Aufnahme hervorsteht.

Die nachfolgende Beschreibung bevorzugter Ausführungsformen der Erfindung dient im Zusammenhang mit der Zeichnung der näheren Erläuterung. Es zeigen:

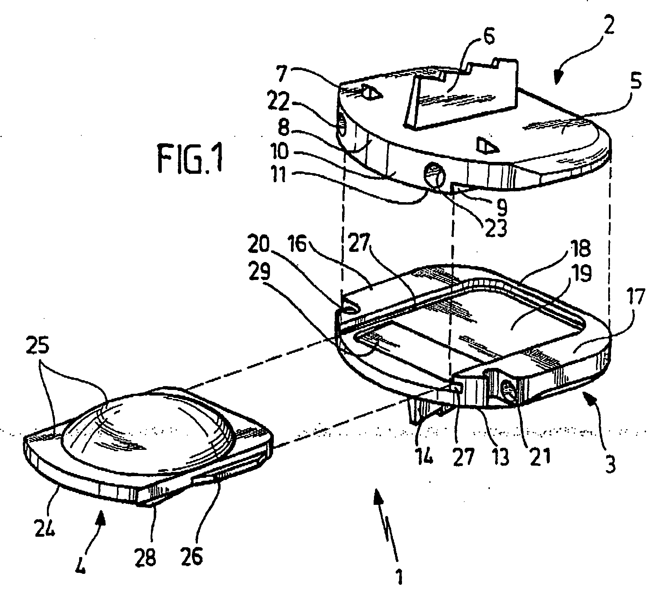

Figur 1 : eine perspektivische Explosionsansicht eines Zwischenwirbelimplantates mit einem Oberteil, einem Unterteil und einem zwischen diese einsetzbaren Gelenkeinsatz ;

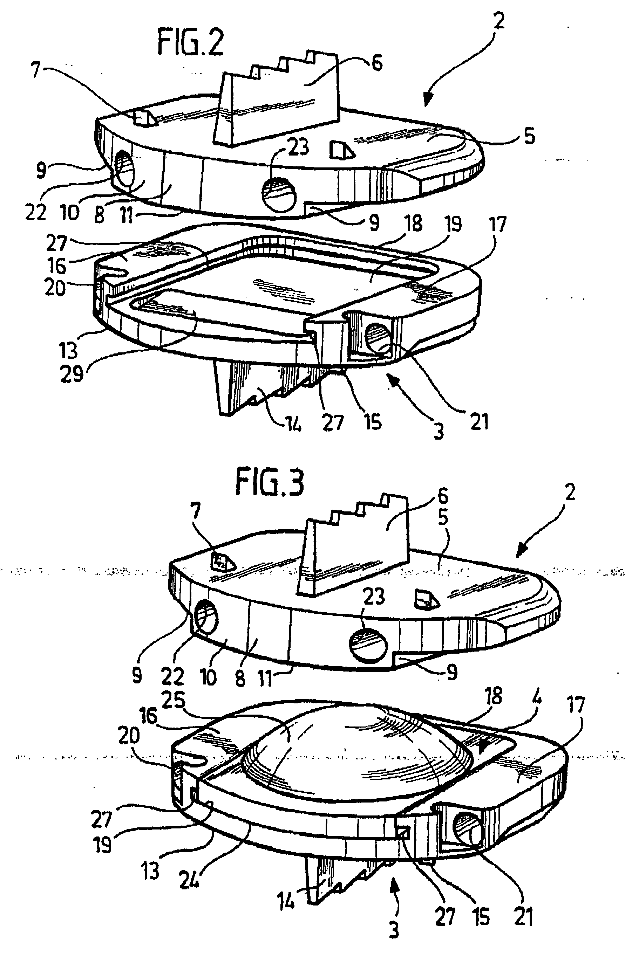

Figur 2 : eine perspektivische Explosionsansicht des Oberteils und des Unterteils des Zwischenwirbelimplantates ohne eingesetzten Gelenkeinsatz ;
Figur 3 : eine Ansicht ähnlich Figur 2 mit in das Unterteil eingeschobenem Gelenkeinsatz ;
Figur 4 : eine perspektivische Ansicht des Oberteils und des Unterteils des Zwischenwirbelimplantates in maximaler gegenseitiger Annäherung ;
Figur 5 : eine Vorderansicht des Zwischenwirbelimplantats der Figur 4;
Figur 6 : eine perspektivische Ansicht des Zwischenwirbelimplantates mit eingesetztem Gelenkeinsatz und
Figur 7 : eine Querschnittsansicht des Zwischenwirbelimplantats der Figur 6.

Das in der Zeichnung dargestellte Zwischenwirbelimplantat 1 umfasst drei Teile, nämlich ein plattenförmiges Oberteil 2, ein plattenförmiges Unterteil 3 und einen weitgehend plattenförmig ausgebildeten Gelenkeinsatz 4,

Das Oberteil 2 ist an seiner Oberseite eben ausgebildet, so dass dadurch eine Stützfläche 5 entsteht, auf der verschiedenartige Vorsprünge 6,7 angeordnet sind, die der Verankerung des Oberteils 2 in einem Wirbelknochen dienen, der mit seiner einem Zwischenwirbelraum zugewandten Endfläche auf der Stützfläche 5 aufliegt.

Das Oberteil 2 hat einen im wesentlichen rechteckigen Querschnitt, wobei in dem dargestellten Ausführungsbeispiel eine Längskante 8 nach außen gebogen ist.

An den beiden Schmalseiten dieses Rechteckes ist die Dicke des plattenförmigen Oberteiles 2 kleiner als im zentralen Bereich, so dass sich längs der schmalen Seiten des Oberteils 2 jeweils parallel zu diesen Kanten verlaufende, nach unten weisende Rücksprünge 9 ausbilden, die zur Außenseite hin offen sind. Zwischen den beiden Rücksprüngen 9 befindet sich der zentrale Bereich des Oberteils 2, der somit eine größere Dicke oder Höhe aufweist und somit einen zwischen den beiden Rücksprüngen 9 ausgebildeten, nach unten weisenden Vorsprung 10 ausbildet. Dieser wird durch eine im wesentlichen parallel zur Stützfläche 5 verlaufende Unterseite 11 begrenzt, in der sich eine kugelige Vertiefung 12 befindet, diese bildet eine Lagerschale aus für den Gelenkeinsatz 4.

Das Unterteil 3 des Zwischenwirbelimplantates 1 ist ebenfalls plattenförmig ausgebildet und weist an seiner Unterseite eine ebene Stützfläche 13 mit Vorsprüngen 14 und 15 auf, die den Vorsprüngen 6 und 7 der Stützfläche 5 entsprechen. Auf der der Stützfläche 13 abgewandten Seite ist die Dicke des Unterteils 3 im zentralen Bereich geringer als in einem außenliegenden Bereich.

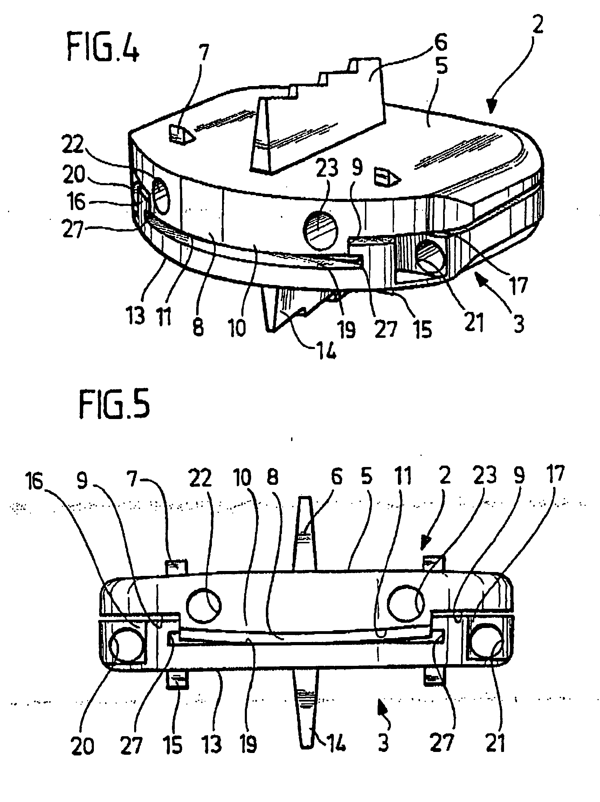

Dieser außenliegende Bereich mit größerer Dicke hat die Form eines U mit zwei parallelen Schenkeln 16,17, die parallel zu den schmalkanten des im Querschnitt ähnlich wie das Oberteil 2 ausgebildeten Unterteiles 3 verlaufen, und mit einem die beiden Schenkel 16 und 17 an einer Seite verbindenden Steg 18. Der von den Schenkeln 16 und 17 und dem Steg 18 eingeschlossene Bereich bildet eine zentrale Vertiefung 19, deren Fläche im wesentlichen der Fläche des zentralen Vorsprunges 10 des Oberteils 2 entspricht, während die Anordnung und die Erstreckung der Schenkel 16 und 17 im wesentlichen der Anordnung und Erstreckung der Rücksprünge 9 am Oberteil 2 entsprechen. Es ist dadurch möglich, Oberteil 2 und Unterteil 3 so aufeinanderzulegen, dass der zentrale Vorsprung 10 des Oberteils 2 in die zentrale Vertiefung 19 eintaucht, während die Schenkel 16 und 17 des Unterteils 3 in die Rücksprünge 9 des Oberteils 2 eintauchen (Figur 4), in dieser Stellung sind Oberteil 2 und Unterteil 3 maximal einander angenähert und weisen eine minimale Bauhöhe auf.

Die Abmessungen sind dabei so gewählt, dass im wesentlichen die jeweiligen Rücksprünge durch die eintauchenden Vorsprünge vollständig ausgefüllt werden.

In die beiden Schenkel 16 und 17 des Unterteils 3 sind von deren freien Enden her parallel zu diesen Schenkeln 16,17 verlaufend Sacklochbohrungen 20 und 21 eingearbeitet, deren Durchmesser im Verhältnis zur Höhe der Schenkel 16,17 relativ groß ist, dieser Durchmesser ist tatsächlich größer als die Dicke oder Höhe des Unterteils 3 im Bereich der zentralen Vertiefung 19.

In den zentralen Vorsprung 10 des Oberteils 2 sind in der Nähe von dessen Seitenkanten Sacklochbohrungen 22 und 23 eingearbeitet, die parallel zu den Sacklochbohrungen 20 und 21 im Unterteil 3 verlaufen. Auch diese Sacklochbohrungen 22 und 23 haben einen relativ großen Durchmesser, der einem wesentlichen Teil der Höhe des Vorsprunges 10 entspricht und größer ist als die Dicke des Oberteils 2 im Bereich der Rücksprünge 9.

Wenn Oberteil 2 und Unterteil 3 in der beschriebenen Weise dicht aneinander anliegen, überlappen sich die Sacklochbohrungen 20 und 21 des Unterteils 3 und die Sacklochbohrungen 22 und 23 des Oberteils 2 in Richtung der Höhe des Zwischenwirbelimplantates 1 zumindest teilweise, wie dies aus der Darstellung der Figuren 4 und 5 deutlich wird.

Die Sacklochbohrungen 20,21,22 und 23 dienen als Aufnahmen für stiftförmige Verlängerungen eines in der Zeichnung nicht dargestellten Handhabungsinstrumentes und bilden somit Angriffselemente für dieses Handhabungsinstrument, welches auf diese Weise getrennt am Oberteil 2 und am Unterteil 3 angreift. Es ist mit diesem Handhabungsinstrument möglich, Oberteil 2 und Unterteil 3 des Zwischenwirbelimplantates 1 in einen Zwischenwirbelraum einzuführen, dabei erleichtert die sehr geringe Bauhöhe des Zwischenwirbelimplantates 1 dieses Einführen, das im wesentlichen ohne große Aufweitung des Zwischenwirbelraumes möglich ist.

Nach dem Einführen des Oberteils 2 und des Unterteils 3 in dieser Weise können die beiden Teile des Zwischenwirbelimplantates 1 aufgespreizt werden, d. h. ihr Abstand wird beispielsweise mit Hilfe des das Oberteil 2 und das Unterteil 3 haltenden Handhabungsinstrumentes vergrößert.

In dieser aufgespreizten Lage von Oberteil 2 und Unterteil 3 ist es möglich, den Gelenkeinsatz 4 zwischen Oberteil 2 und Unterteil 3 einzuschieben.

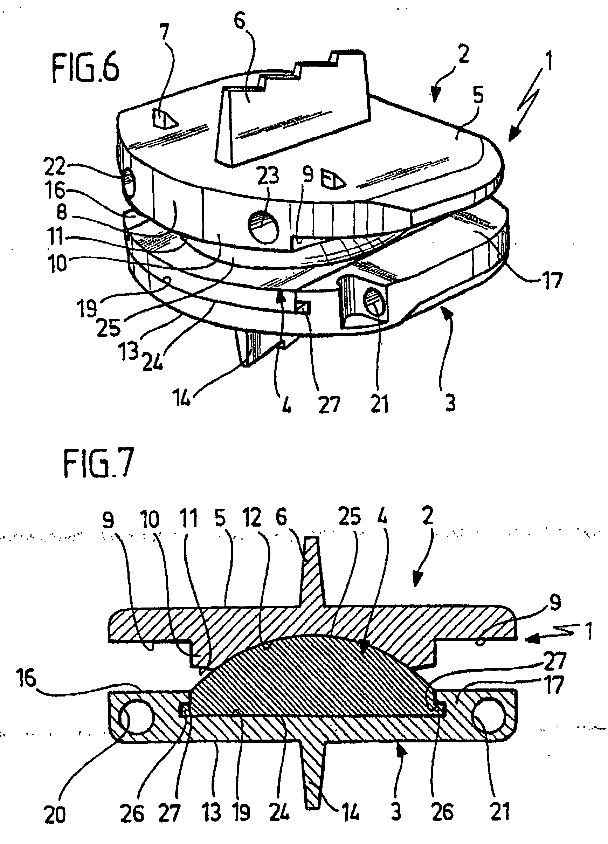

Dieser Gelenkeinsatz 4 ist im wesentlichen in Form einer Platte aufgebaut, die eine ebene Unterseite 24 und eine kugelig aufgewölbte Oberseite 25 aufweist. Die Außenabmessungen des plattenförmigen Gelenkeinsatzes 4 entsprechen denen der zentralen Vertiefung 19 im Unterteil 3, so dass der Gelenkeinsatz 4 diese Vertiefung ausfüllend in diese eingeschoben werden kann, und zwar von der Seite her, auf die sich die Sacklochbohrungen 20,21,22,23 öffnen. Dabei greifen Führungsleisten 26 an den Seitenkanten des Gelenkeinsatzes 4 in entsprechende Führungsnuten 27 in den Schenkeln 16,17 ein, so dass eine Einschubführung für den Gelenkeinsatz 4 gebildet wird, die diesen nach dem Einsetzen im Unterteil 3 festlegen. Der eingeschobene Gelenkeinsatz 4 füllt nach dem Einschieben die Vertiefung 19 aus und steht mit seiner kugelig gewölbten Oberseite 25 nach oben über die Oberseite des Unterteiles 3 hervor, die kugelige Oberseite 25 taucht dabei komplementär in die kugelig gewölbte Vertiefung 12 an der Unterseite des Vorsprunges 10 ein und bildet dort mit Oberteil 2 ein Kugelgelenk aus, welches eine gewisse Verschwenkbarkeit des Oberteils 2 gegenüber dem Unterteil 3 ermöglicht (Figur 7).

Der Gelenkeinsatz 4 kann an seiner ebenen Unterseite 24 einen Rastvorsprung 28 tragen, der beim Einschieben des Gelenkeinsatzes 4 in das Unterteil 3 elastisch in eine Rastausnehmung 29 einrastet, die sich am Boden der Vertiefung 19 befindet ; dadurch wird der Gelenkeinsatz 4 auch in Einschubrichtung in der Vertiefung 19 festgelegt.

Oberteil 2 und Unterteil 3 sind vorzugsweise aus körperverfraglichem Metall hergestellt, beispielsweise aus Titan, während der Gelenkeinsatz 4 vorzugsweise aus einem ebenfallskörperverträglichen Kunststoffmaterial besteht, beispielsweise aus Polyethylen. Diese Stützflächen 5 bzw. 13 können besonders knochenverträglich ausgebildet sein, beispielsweise kann diese Fläche durch eine Beschichtung aufgerauht werden, so dass sich eine optimale Verankerung mit dem benachbarten Knochenmaterial ergibt.

## Patentansprüche

1. Zwischenwirbelimplantat mit einem eine Stützfläche (5) für einen Wirbelkörper aufweisenden ersten Teil (2) und einem eine Stützfläche (13) für einen benachbarten Wirbelkörper aufweisenden zweiten Teil (3), an denen jeweils von einer Seite des Zwischenwirbelimplantats (1) her zugängliche Angriffselemente (20, 21, 22, 23) für eine Handhabungsinstrument angeordnet sind, wobei zwischen dem ersten Teil (2) und dem zweiten Teil (3) eine kugelige Stützfläche (25) vorgesehen ist, die zu einer Verschwenkbarkeit des ersten und des zweiten Teils (2, 3) relativ zueinander führt, weiterhin erste Vorsprünge (6, 14) vorgesehen sind, mit welchen das erste Teil (2) und das zweite Teil (3) jeweils an einem Wirbelkörper verankerbar sind, **dadurch gekennzeichnet, dass** sowohl die Stützfläche (5) des ersten Teil (2) als auch die Stützfläche (13) des zweiten Teils (3) nur jeweils einen Vorsprung (6, 14) als Verankerung in der Mitte zwischen Schmalseiten des jeweiligen Teils (2, 3) aufweisen, deren Höhe senkrecht zu den Stützflächen (5, 13) größer als ihre Breite in einer Querrichtung zwischen den Schmalseiten ist, wobei sich die Vorsprünge mittig in Längsrichtung zwischen den Schmalseiten über einen Großteil der jeweiligen Stützfläche (5, 13) erstrecken.

2. Zwischenwirbelimplantat nach Anspruch 1, **dadurch gekennzeichnet, dass** an den Stützflächen (5, 13) weitere Vorsprünge (7, 15) mit geringerer Höhe und geringerer Längserstreckung als die ersten Vorsprünge (6, 14) vorgesehen sind.

3. Zwischenwirbelimplantat nach Anspruch 1 oder 2, **dadurch gekennzeichnet, dass** das erste Teil (2) und das zweite Teil (3) jeweils einen im wesentlichen rechteckigen Querschnitt besitzen, wobei die Längskanten (8) länger sind als die Schmalseiten.

4. Zwischenwirbelimplantat nach Anspruch 3, **dadurch gekennzeichnet, dass** die Längskanten (8) nach außen gebogen sind.

5. Zwischenwirbelimplantat nach einem der vorhergehenden Ansprüche, **dadurch gekennzeichnet, dass** die kugelige Stützfläche (25) des ersten Teils (2) und des zweiten Teils (3, 4) eine Gelenkabstützung (4) bilden.

6. Zwischenwirbelimplantat nach Anspruch 5, **dadurch gekennzeichnet, dass** die Gelenkabstützung eine kugelige Stützfläche (25) an einem der Teile (3, 4) aufweist, die an einer passenden kugeligen Fläche (12) an dem anderen Teil (2) abgestützt ist.

7. Zwischenwirbelimplantat nach einem der vorhergehenden Ansprüche, **dadurch gekennzeichnet, dass** der erste Teil (2) und der zweite Teil (3) je eine Aufnahme (12, 15) für einen Gelenkeinsatz aufweisen.

8. Zwischenwirbelimplantat nach Anspruch 7, **dadurch gekennzeichnet, dass** bei fehlendem Gelenkeinsatz (4) das erste Teil (2) und das zweite Teil (3) verschachtelt angeordnet sind.

9. Zwischenwirbelimplantat nach Anspruch 7, **dadurch gekennzeichnet, dass** der Gelenkeinsatz (4) in dem ersten Element (3) eingerastet ist und die kugelige Stützfläche (25) aufweist, die in eine entsprechend kugelige Aufnahme (12) an dem ersten Teil (2) eingreift.

10. Zwischenwirbelimplantat nach einem der vorhergehenden Ansprüche, **dadurch gekennzeichnet, dass** wenigstens die ersten Verankerungen (6, 14) der beiden Teile (2, 3) derart angeordnet sind, dass sie jeweils in eine Nut des zugehörigen Wirbelkörpers eingreifen, wenn das Implantat (1) in den Zwischenwirbelraum bewegt wird.

11. Zwischenwirbelimplantat nach einem der vorhergehenden Ansprüche, **dadurch gekennzeichnet, dass** beide Teile (2, 3, 4) des Implantats gemeinsam in einen Zwischenwirbelraum implantierbar sind.

12. Zwischenwirbelimplantat nach einem der vorhergehenden Ansprüche, **dadurch gekennzeichnet, dass** die ersten Verankerungen (6, 14) mehrere Zähne aufweisen.

## Claims

1. An intervertebral implant comprising a first part (2) having a supporting surface (5) for a vertebrae and a second part (3) having a supporting surface (13) for an adjacent vertebrae, at each of which engagement means (20, 21, 22, 23) being accessible from one side for a handling instrument are provided, wherein a spherical surface (12,25) is provided between the first part (2) and the second part (3) for allowing a joint movement of the first and second part relative to each other, and furthermore first protrusions (6) are provided by which the first and the second part each can be anchored in a vertebrae, **characterized in that** both the supporting surface (5) of the first part (2) and the supporting surface (13) of the second part (3) in their center between narrow sides each have only one protrusion (6,14)for anchoring having a height in a direction perpendicular to the supporting surface (5) being greater than their width in a transversal direction between the narrow sides, where said protrusions extend lengthwise in the center along the greater part of the respective supporting surface (5,13).

2. An intervertebral implant according to claim 1, **characterized in that** further protrusions (7,15) of minor height and length are provided on the supporting surfaces (5,13).

3. An intervertebral implant according to claim 1 or 2 **characterized in that** the first part (2) and the second part each have a substantially rectangular transverse section, where the longitudinal edges (8) are longer than the narrow sides.

4. An Intervertebral implant according to claim 3, **characterized in that** the longitudinal edges (8) are curved to the outside.

5. An intervertebral implant according to one of the preceding claims, **characterized in that** the spherical surfaces (12, 25) of the first part (2) and the second part (3, 4) form a universal engagement (4).

6. An intervertebral implant according to claim 5, **characterized in that** the universal engagement includes a spherical surface (25) on one of the parts (3, 4) operatively engaged with a mating spherical.surface (12) on the other part (2).

7. An intervertebral implant according to one of the preceding claims, **characterized in that** the first part (2) and the second part (3) each have a seating (12,19) for a pivot insert (4).

8. An intervertebral implant according to claim 7, **characterized in that**, in the absence of the pivot insert (4), the first part (2) nests into the second part (3).

9. An intervertebral implant according to claim 5, **characterized in that** the pivot insert (4) snap fits into the first piece (3) and comprises said spherical surface (25) engaging the respective spherical seating of the first part (2).

10. An intervertebral implant according to one of the preceding claims, **characterized in that** at least the first protrusions (6, 14) of both parts (2, 3, 4)are positioned such that they engage a groove in its respective vertebrae as the implant (1) is moved into the intervertebral space.

11. An intervertebral implant according to one of the preceding claims, **characterized in that** the two parts (2, 3, 4) of the implant are adapted for implantation together into an intervertebral space.

12. An intervertebral implant according to one of the preceding claims, **characterized in that** the first protrusions (6, 14) comprise a series of teeth.

## Revendications

1. Implant intervertébral comprenant une première partie (2) comportant une surface d'appui (5) pour un corps de vertèbre et une seconde partie (3) comportant une surface d'appui (13) pour un corps de vertèbre voisin, sur lesquelles sont respectivement montés des élément de mise en prise (20, 21, 22, 23) d'un instrument de manipulation respectivement accessibles à partir d'un côté de l'implant intervertébral (1), entre la première partie (2) et la seconde partie (3) étant prévue une surface d'appui sphérique (25) qui permet une liberté de pivotement de la première et de la seconde partie (2, 3) relativement l'une à l'autre, des premières saillies (6, 14) étant en outre prévues, ces premières saillies (6, 14) permettant respectivement d'ancrer la première partie (2) et la seconde partie (3) sur un corps de vertèbre,
**caractérisé en ce que**
la surface d'appui (5) de la première partie (2) ainsi que la surface d'appui (13) de la seconde partie (3) ne comportent respectivement qu'une seule saillie (6, 14) constituant un élément d'ancrage respectivement située au milieu de ces surfaces d'appui entre les côtés étroites des parties (2, 3) respectives, et dont la hauteur perpendiculairement aux surfaces d'appui (5, 13) est supérieure à la largeur dans la direction transversale entre les côtés étroites, les saillies étant centrées et s'étendant en direction longitudinale entre les côtés étroites sur une grande partie des surfaces d'appui (5, 13) respectives.

2. Implant intervertébral conforme à la revendication 1,
**caractérisé en ce que**
sur les surfaces d'appui (5, 13) sont prévues d'autres saillies (7, 15) ayant une plus faible hauteur et une extension longitudinale plus faible que les premières saillies (6, 14).

3. Implant intervertébral conforme à la revendication 1 ou 2,
**caractérisé en ce que**
la première partie (2) et la seconde partie (3) ont respectivement une section essentiellement rectangulaire, les arêtes longitudinales (8) étant plus longues que les côtés étroites.

4. Implant intervertébral conforme à la revendication 3,
**caractérisé en ce que**
les arêtes longitudinales (8) sont courbées vers l'extérieur.

5. Implant intervertébral conforme à l'une des revendications précédentes,
**caractérisé en ce que**
les surfaces d'appui sphériques (25) de la première partie (2) et de la seconde partie (3, 4) forment un appui articulé (4).

6. Implant intervertébral conforme à la revendication 5,
**caractérisé en ce que**
l'appui articulé comporte une surface d'appui sphérique (25) sur l'une des parties (3, 4) qui s'appuie sur une surface sphérique correspondante (12) de l'autre partie (2).

7. Implant intervertébral conforme à l'une des revendications précédentes,
**caractérisé en ce que**
la première partie (2) et la seconde partie (3) comportent chacune un logement de réception (12, 15) d'un insert d'articulation.

8. Implant intervertébral conforme à la revendication 4,
**caractérisé en ce qu'**
en l'absence d'insert d'articulation (4) la première partie (2) et la seconde partie (3) sont imbriquées l'une dans l'autre.

9. Implant intervertébral conforme à la revendication 7,
**caractérisé en ce que**
l'insert d'articulation (4) est enclipés dans le premier élément (3) et comporte la surface d"appui sphérique (25) qui vient en prise dans un logement de réception sphérique (12) correspondant de la première partie (2).

10. Implant intervertébral conforme à l'une des revendications précédentes,
**caractérisé en ce qu'**
au moins les premiers éléments d'ancrage (6, 14) des deux parties (2, 3) sont montés de sorte qu'il viennent respectivement en prise dans une rainure du corps de vertèbre correspondant lorsque l'implant (1) est déplacé dans l'espace intervertébral.

11. Implant intervertébral conforme à l'une des revendications précédentes,
**caractérisé en ce que**
les deux parties (2, 3, 4) de l'implant peuvent être implantées en commun dans un espace intervertébral.

12. Implant intervertébral conforme à l'une des revendications précédentes,
**caractérisé en ce que**
les premiers éléments d'ancrage (6, 14) comportent plusieurs dents.
